# EUROPEAN PATENT APPLICATION

(11) **EP 1 964 517 A1**
(43) Date of publication of application: **03.09.2008**
(21) Application number: 08003444.0
(22) Date of filing: 26.02.2008
(51) Int. Cl.: A61B 8/00, G01S 7/52, G01S 15/89

(54) **Ultrasound system providing a power save mode**

(30) Priority: 27.02.2007 KR 20070019317
(71) Applicant: MEDISON CO., LTD., Kangwon-do 250-870 (KR)
(72) Inventor: Choi, Seok Won, Gangnam-gu Seoul 135-280 (KR)
(74) Representative: Schmid, Wolfgang

(57) **Abstract**

The present invention is directed to an ultrasound system providing a power save mode. The ultrasound system includes: a power supply unit (110) to output a supply power; an ultrasound diagnosis unit (130) to transmit ultrasound signals to a target object and receive ultrasound echo signals reflected from the target object, the ultrasound diagnosis unit being configured to form an ultrasound image based on the ultrasound echo signals; a switch unit (120) coupled between the ultrasound diagnosis unit and the power supply unit; and a control unit (140) to control the switch unit for providing the supply power from the power supply unit to the ultrasound diagnosis unit depending on an operation state of the ultrasound diagnosis unit. The power supply unit individually supplies a power to the ultrasound diagnosis unit and the control unit.

## Description

The present application claims priority from Korean Patent Application No. 10-2007-0019317 filed on February 27, 2007, the entire subject matter of which is incorporated herein by reference.

### BACKGROUND OF THE INVENTION

### [Technical Field]

The present invention generally relates to ultrasound systems, and more particularly to an ultrasound system providing a power save mode.

### [Background Art]

An ultrasound system has become an important and popular diagnostic tool due to its non-invasive and non-destructive nature. Modern high-performance ultrasound imaging diagnostic systems and techniques are commonly used to produce two- or three-dimensional images of internal features of patients.

The ultrasound system generally uses a probe containing an array of transducer elements to transmit and receive ultrasound signals. The ultrasound system forms an image of human internal tissues by electrically exciting transducer elements to generate ultrasound signals that travel into the body. Echoes reflected from tissues and organs return to the transducer element and are converted into electrical signals, which are amplified and processed to produce an ultrasound image data.

In the conventional ultrasound system, power is continuously supplied to the ultrasound system when the user does not manipulate the ultrasound system for a constant time. As a result, an excessive amount of heat is generated during the operation of the ultrasound system. In order to operate the ultrasound system, the heat should be cooled. Thus, the user or patient may feel uncomfortable due to a noise generated from a cooling device as well as the heat. Further, unnecessary power consumption is caused and the lifetime of the ultrasound system may be shortened.

### BRIEF DESCRIPTION OF THE DRAWINGS

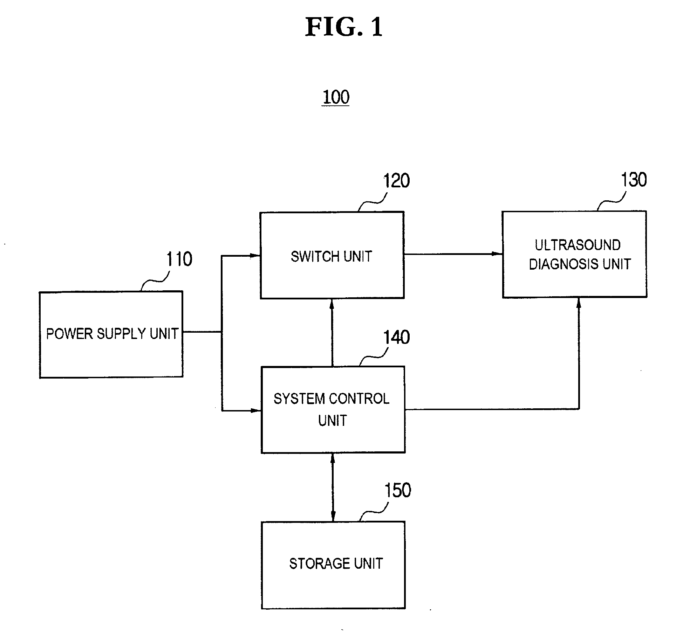

FIG. 1 is a block diagram showing an ultrasound system constructed in accordance with one embodiment of the present invention.

### DETAILED DESCRIPTION OF THE INVENTION

FIG. 1 is a block diagram showing an ultrasound system constructed in accordance with one embodiment of the present invention. As shown in Fig. 1, the ultrasound system 100 includes a power supply unit 110, a switch unit 120, an ultrasound diagnosis unit 130, a system control unit 140 and a storage unit 150. The ultrasound system 100 may further include an input unit (not denoted) for receiving an input from a user. The input unit may include a control panel, a mouse, a trackball, a foot switch, etc.

The power supply unit 110 individually supplies power to the ultrasound diagnosis unit 130 and the system control unit 140. The switch unit 120 is coupled between the power supply unit 110 and the ultrasound diagnosis unit 130. The switch unit 120 becomes an on or off state under the control of the system control unit 140, thereby connecting or disconnecting the power supply to the ultrasound diagnosis unit 130.

The ultrasound diagnosis unit 130 may include a plurality of elements such as a probe, a beam former, a signal processor, etc. for forming an ultrasound image. Under the control of the system control unit 140, the ultrasound diagnosis unit 130 transmits ultrasound signals to a target object and receives ultrasound echo signals reflected from the target object. The ultrasound diagnosis unit 130 may form the ultrasound image based on the ultrasound echo signals. The storage unit 150 may store operation information necessary to operate each element of the ultrasound diagnosis unit 130.

If a user turns on the ultrasound system 100, then the power supply unit 110 supplies power to the system control unit 140 so that the system control unit 140 is initialized and booted. The system control unit 140 generates a first control signal and transmits the first control signal to the switch unit 120. The switch unit 120 is switched to an on state in response to the first control signal and the power is supplied to the ultrasound diagnosis unit 130. If power is supplied to the ultrasound diagnosis unit 130, then the system control unit 140 reads out the operation information from the storage unit 150 and transmits the operation information to the ultrasound diagnosis unit 130.

The system control unit 140 continuously checks whether or not the ultrasound diagnosis unit 130 operates. The operation of the ultrasound diagnosis unit 130 may include transmission/reception of the ultrasound signals, signal processing of the received signals, manipulation of the input unit, etc. If it is determined that the ultrasound diagnosis unit 130 does not operate for a predetermined time, then the system control unit 140 extracts current state operation information of the ultrasound diagnosis unit 130. The state information may be operation information on each element state of the ultrasound diagnosis unit 130 and image setup information. The extracted state operation information of the ultrasound diagnosis unit 130 is stored in the storage unit 150. Thereafter, the system control unit 140 generates a second control signal and transmits the second control signal to the switch unit 120. The switch unit 120 is switched to an off state in response to the second control signal such that the power supply to the ultrasound diagnosis unit 130 is disconnected. Then, the ultrasound system 100 goes into a power-save mode in accordance with the present invention.

In the power-save mode, the power is merely supplied to the system control unit 140 and the input unit. The system control unit 140 keeps checking whether or not the input unit is manipulated in the power-save mode. If it is determined that the input unit has been manipulated, then the system control unit 140 generates the first control signal and transmits the first control signal to the switch unit 120. The switch unit 120 is turned into an on state in response to the first control signal and the power from the power supply unit 110 is supplied to the ultrasound diagnosis unit 130. The system control unit 140 reads out the operation state information stored in the storage unit 150 and transmits the information to the ultrasound diagnosis unit 130 such that the state of the ultrasound diagnosis unit 139 is restored to an operation state prior to the power-save mode.

As mentioned above, since the ultrasound system goes into the power-save mode when the ultrasound system does not operate for a predetermined time in accordance with the present invention, power consumption of the ultrasound system can be reduced. Also, since unnecessary operations of the cooling device and the ultrasound diagnosis unit are not needed in the power-save mode, noise and heat radiation may be reduced and lifetime may be increased.

In accordance with one embodiment of the present invention, there is provided an ultrasound system, including: a power supply unit; an ultrasound diagnosis unit to transmit ultrasound signals to a target object and receive ultrasound echo signals reflected from the target object, the ultrasound diagnosis unit being configured to form an ultrasound image based on the ultrasound echo signals; a switch unit coupled between the ultrasound diagnosis unit and the power supply unit; and a control unit to control the switch unit for connecting or disconnecting power supply to the ultrasound diagnosis unit by checking whether or not the ultrasound diagnosis unit operates, wherein the power supply unit individually supplies a power to the ultrasound diagnosis unit and the control unit.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a power supply unit to output a supply power;
an ultrasound diagnosis unit to transmit ultrasound signals to a target object and receive ultrasound echo signals reflected from the target object, the ultrasound diagnosis unit being configured to form an ultrasound image based on the ultrasound echo signals;
a switch unit coupled between the ultrasound diagnosis unit and the power supply unit; and
a control unit to control the switch unit for providing the supply power from the power supply unit to the ultrasound diagnosis unit depending on an operation state of the ultrasound diagnosis unit,
wherein the power supply unit individually supplies a power to the ultrasound diagnosis unit and the control unit.

2. The ultrasound system of Claim 1, wherein the control unit checks whether or not the ultrasound diagnosis unit is in operation and generates a first control signal to allow the switch unit to be an off state when the ultrasound diagnosis unit is not in operation for a predetermined time.

3. The ultrasound system of Claim 2, wherein the control unit extracts operation information of the ultrasound diagnosis unit before the off state of the switch unit.

4. The ultrasound system of Claim 3, further comprising a storage unit to store the operation information.

5. The ultrasound system of Claim 4, further comprising an input unit to receive an input from a user.

6. The ultrasound system of Claim 5, wherein the control unit checks whether or not the input unit receives the input to generate a second control signal for allowing the switch unit to be an on state and transmitting the operation information stored in the storage unit to the ultrasound diagnosis unit in response to the input of the input unit.
